# EUROPEAN PATENT SPECIFICATION

(11) **EP 3 941 287 B1**
(45) Date of publication and mention of the grant of the patent: **27.11.2024**
(21) Application number: 20716393.2
(22) Date of filing: 13.03.2020
(51) Int. Cl.: A61M 11/04, A61M 15/06, A24F 40/00, A61M 16/00, A61K 9/00, H05B 3/46, A24F 40/40

(54) **SMOKING SUBSTITUTE SYSTEM**
RAUCHERSATZSYSTEM
SYSTÈME DE SUBSTITUTION DU TABAC

(30) Priority: 22.03.2019 EP 19020203
(43) Date of publication of application: 26.01.2022
(60) Divisional application: 24207761.8
(73) Proprietor: Imperial Tobacco Limited, Bristol, BS3 2LL (GB)
(72) Inventor: FERRIE, Kate, Liverpool Merseyside L24 9HP (GB); LORD, Chris, Liverpool Merseyside L24 9HP (GB); SHENTON, Ross, Liverpool Merseyside L24 9HP (GB); LOMAS, Pete, Liverpool Merseyside L24 9HP (GB)
(74) Representative: Mewburn Ellis LLP
(86) International application number: PCT/EP2020/056792
(87) International publication number: WO 2020/193189

(56) References cited:
- WO-A1-2015/100361
- WO-A1-2017/051150
- WO-A1-2018/099999
- WO-A1-2018/122389
- CN-U- 206 542 921
- US-A1- 2013 192 615

## Description

### TECHNICAL FIELD

The present invention relates to a smoking substitute system and particularly, although not exclusively, to a smoking substitute system comprising a smoking substitute device and an aerosol-forming article.

### BACKGROUND

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful by-products. There have been proposed various smoking substitute systems (or "substitute smoking systems") in order to avoid the smoking of tobacco (see e.g. WO 2018/099999, CN 206 542 921, WO 2015/100361 and WO 2018/122389).

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or flavourings without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products. Some smoking substitute systems use smoking substitute articles (also referred to as a "consumables") that are designed to resemble a traditional cigarette and are cylindrical in form with a mouthpiece at one end.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories.

There are a number of different categories of smoking substitute systems, each utilising a different smoking substitute approach.

One approach for a smoking substitute system is the so-called Heated Tobacco ("HT") approach in which tobacco (rather than an "e-liquid") is heated or warmed to release vapour. HT is also known as "heat not burn" ("HNB"). The tobacco may be leaf tobacco or reconstituted tobacco. The vapour may contain nicotine and/or flavourings. In the HT approach the intention is that the tobacco is heated but not burned, i.e. the tobacco does not undergo combustion.

A typical HT smoking substitute system may include a device and a consumable. The consumable may include the tobacco material. The device and consumable may be configured to be physically coupled together. In use, heat may be imparted to the tobacco material by a heating element of the device, wherein airflow through the tobacco material causes components in the tobacco material to be released as vapour. A vapour may also be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the consumable (entrained in the airflow) from the location of vaporisation to an outlet of the consumable (e.g. a mouthpiece), the vapour cools and condenses to form an aerosol for inhalation by the user. The aerosol will normally contain the volatile compounds.

In HT smoking substitute systems, heating as opposed to burning the tobacco material is believed to cause fewer, or smaller quantities, of the more harmful compounds ordinarily produced during smoking. Consequently, the HT approach may reduce the odour and/or health risks that can arise through the burning, combustion and pyrolytic degradation of tobacco.

Some of the currently available HT smoking substitute devices typically require an airflow to enter the device through openings provided on a housing of the device. Such opening may be provided on a major surface of the housing, which may be susceptible to blockage by the user's fingers as the user holds onto the device. Therefore in some other prior art devices, air inlets are provided at a location away from the major surface of the device, in order to reduce the likelihood of such inadvertent blockage. For example, air inlets in some devices are provided at a tip of a cap and thus an airflow is required to flow through a length of air flow annulus in the cap before it converges towards the consumable. Such arrangement may increase the draw resistance during a puff, and in some cases it may even limit the amount of airflow that is available for entraining the vapour released from the tobacco. Furthermore, such arrangement may result in a diffused air supply to the heating element, thus impacting heater transfer within the aerosol-forming article.

There may be a need for improved design of smoking substitute systems, in particular HT smoking substitute systems, to enhance the user experience, to improve aerosol generation and Total Particulate Matter (TPM) output of the aerosol of the HT smoking substitute system.

The present disclosure has been devised in the light of the above considerations.

### SUMMARY OF THE INVENTION

At its most general, the present invention relates to a smoking substitute device with an improved air inlet that may reduce the likelihood of inadvertent blockage by a user. Furthermore, the air inlet may allow airflow to be directed at a base of the heating element, and thereby it may improve aerosol generation and Total Particulate Matter (TPM) output of the aerosol of the HT smoking substitute system.

According to a first aspect of the present invention, there is provided a smoking substitute device. The smoking substitute device comprises a housing; and a cap configured to engage with the housing and thereby defines an air inlet between the cap and the housing; wherein the air inlet is configured to facilitate an airflow to enter into the housing.

The cap may be slideable along a longitudinal axis of the device, between a first position where at least a peripheral portion of the cap is positioned adjacent to a corresponding peripheral portion of the housing and a second position where the cap is positioned away, but not necessary detached, from the housing.

By providing the smoking substitute device comprising an air inlet defined between the cap and the housing, it may advantageously prevent the user from inadvertently blocking said air inlet. This is because the interface between the cap and housing is positioned at an edge or a peripheral portion of the housing and therefore the user may be less likely to hold onto the device by said interface.

Optional features will now be set out. These are applicable singly or in any combination with any aspect.

The air inlet extends in a direction transverse to longitudinal axis of the housing. Advantageously, such arrangement may allow the air inlet to position along an edge or a peripheral portion of the housing or the cap.

The device further comprises a heating element, and the air inlet provides airflow into the housing underneath the heating element.

Optionally, the air inlet is located adjacent to a heating element of the housing. Optionally, the air inlet facilitates the airflow to flow towards a base of the heating element. For example, the cap may be configured to receive an aerosol-forming article and whereby during use, the heating element may be configured to fully penetrate into said article. Conveniently, the base of the heater may correspond to an end of the cap when the cap is engaged with the housing. Therefore the air inlet formed between the cap and the housing may be positioned immediately adjacent to the heating element. Advantageously, such arrangement may reduce draw resistant offered by a shorter air flow path, as well as increasing the amount of heat convection by directing the air flow towards the base of the heating element, and thereby it may improve the quality of aerosol generation and Total Particulate Matter (TPM) output of the aerosol.

Optionally, the air inlet is defined by a gap formed between the cap and the housing when the cap is engaged with the housing. More specifically, when the cap is engaged with the housing, at least a portion of the cap is spaced from the housing to form such gap. For example, the device may comprise a stop to prevent the cap from abutting the housing so as to define such gap.

Optionally, the cap and/or housing comprises a notch or indentation formed on a respective edge of the cap and/or housing, wherein the notch or indentation on the cap and/or housing forms the air inlet. As such when the cap is engaged with the housing, a portion of the cap may abut the housing and airflow may enter the device through the notch.

Optionally, the air inlet comprises a slit or a through hole.

Optionally, the smoking substitute device comprises a Heat Not Burn (HNB) device.

According to a second aspect of the present invention, there is provided a smoking substitute device comprising a housing and an electrical connection disposed in the housing. The electrical connection comprises an air inlet, to facilitate flow of air into the housing.

For example, the electrical connection may be a socket for receiving an electrical terminal. The electrical connection may comprise an opening that forms the air inlet, which in turn may be arranged to be in fluid communication with the heating element. Therefore an airflow may enter the housing via said air inlet at the electrical connection. The air inlet may remain open even if the electrical connection is engaged with or receiving a corresponding electrical terminal. Advantageously, the provision of an air inlet at said electrical connection may reduce the likelihood of a user blocking said air inlet because the user is not likely to hold onto the device by the electrical connection.

Optionally, the housing comprises a first end engageable with a cap and a second end opposite to the first end, wherein the electrical connection forms on the second end of the device. Advantageously, by locating the electrical connection towards an end of the device, it may reduce the likelihood of user blocking said air inlet because the user is not likely to hold onto the device by its end.

Optionally, the electrical connection is provided at the housing at a position adjacent to the cap. Advantageously, such arrangement may significantly reduce the length of the air flow path and therefore it may reduce draw resistant and thereby it may improve the quality of aerosol generation and Total Particulate Matter (TPM) output of the aerosol.

Optionally, the air inlet at the electrical connection facilitates the airflow to flow towards a base of a heating element of the housing. Advantageously, such arrangement may increase the amount of heat convection by directing the air flow towards the base of the heating element, and thereby it may promote the aerosol generation and increases TPM output.

Optionally, the electrical connection comprises a Universal Serial Bus (USB) connection. Optionally, the electrical connection comprises a USB socket having the air inlet defined therein.

Optionally, the smoking substitute device comprises a Heat Not Burn (HNB) device.

The smoking substitute device (hereinafter referred as device), may comprise a housing. A first end of the housing may be configured for engagement with a cap, wherein the cap may be configured to receive an aerosol-forming article. For example, the housing may be configured for engagement with a heated tobacco (HT) consumable (or heat-not-burn (HNB) consumable). The terms "heated tobacco" and "heat-not-burn" are used interchangeably herein to describe a consumable that is of the type that is heated rather than combusted (or are used interchangeably to describe a device for use with such a consumable). The device may comprise a cavity that is configured for receipt of at least a portion of the consumable (i.e. for engagement with the consumable). The aerosol-forming article may be of the type that comprises an aerosol former (e.g. carried by an aerosol-forming substrate).

The device may comprise a heater for heating the aerosol-forming article. The heater may comprise a heating element, which may be in the form of a rod that extends from the housing of the device. The heating element may extend from the end of the housing that is configured for engagement with the aerosol-forming article.

The heater (and thus the heating element) may be rigidly mounted to the housing. The heating element may be elongate so as to define a longitudinal axis and may, for example, have a transverse profile (i.e. transverse to a longitudinal axis of the heating element) that is substantially circular (i.e. the heating element may be generally cylindrical). Alternatively, the heating element may have a transverse profile that is rectangular (i.e. the heater may be a "blade heater"). The heating element may alternatively be in the shape of a tube (i.e. the heater may be a "tube heater"). The heating element may take other forms (e.g. the heating element may have an elliptical transverse profile). The shape and/or size (e.g. diameter) of the transverse profile of the heating element may be generally consistent for the entire length (or substantially the entire length) of the heating element.

The heating element may be between 15 mm and 25 mm long, e.g. between 18 mm and 20 mm long, e.g. around 19 mm long. The heating element may have a diameter of between 1.5 mm and 2.5 mm, e.g. a diameter between 2 mm and 2.3 mm, e.g. a diameter of around 2.15 mm.

The heating element may be formed of ceramic. The heating element may comprise a core (e.g. a ceramic core) comprising Al2O3. The core of the heating element may have a diameter of 1.8 mm to 2.1 mm, e.g. between 1.9 mm and 2 mm. The heating element may comprise an outer layer (e.g. an outer ceramic layer) comprising AI2O3. The thickness of the outer layer may be between 160 µm and 220 µm, e.g. between 170 µm and 190 µm, e.g. around 180 µm. The heating element may comprise a heating track, which may extend longitudinally along the heating element. The heating track may be sandwiched between the outer layer and the core of the heating element. The heating track may comprise tungsten and/or rhenium. The heating track may have a thickness of around 20 µm.

The heating element may be located in the cavity (of the device), and may extend (e.g. along a longitudinal axis) from an internal base of the cavity towards an opening of the cavity. The length of the heating element (i.e. along the longitudinal axis of the heater) may be less than the depth of the cavity. Hence, the heating element may extend for only a portion of the length of the cavity. That is, the heating element may not extend through (or beyond) the opening of the cavity.

The heating element may be configured for insertion into an aerosol-forming article (e.g. a HT consumable) when an aerosol-forming article is received in the cavity. In that respect, a distal end (i.e. distal from a base of the heating element where it is mounted to the device) of the heating element may comprise a tapered portion, which may facilitate insertion of the heating element into the aerosol-forming article. The heating element may fully penetrate an aerosol-forming article when the aerosol-forming article is received in the cavity. That is, the entire length, or substantially the entire length, of the heating element may be received in the aerosol-forming article.

The heating element may have a length that is less than, or substantially the same as, an axial length of an aerosol-forming substrate forming part of an aerosol-forming article (e.g. a HT consumable). Thus, when such an aerosol-forming article is engaged with the device, the heating element may only penetrate the aerosol-forming substrate, rather than other components of the aerosol-forming article. The heating element may penetrate the aerosol-forming substrate for substantially the entire axial length of the aerosol forming-substrate of the aerosol-forming article. Thus, heat may be transferred from (e.g. an outer circumferential surface of) the heating element to the surrounding aerosol-forming substrate, when penetrated by the heating element. That is, heat may be transferred radially outwardly (in the case of a cylindrical heating element) or e.g. radially inwardly (in the case of a tube heater).

Where the heater is a tube heater, the heating element of the tube heater may surround at least a portion of the cavity. When the portion of the aerosol-forming article is received in the cavity, the heating element may surround a portion of the aerosol-forming article (i.e. so as to heat that portion of the aerosol-forming article). In particular, the heating element may surround an aerosol forming substrate of the aerosol-forming article. That is, when an aerosol-forming article is engaged with the device, the aerosol forming substrate of the aerosol-forming article may be located adjacent an inner surface of the (tubular) heating element. When the heating element is activated, heat may be transferred radially inwardly from the inner surface of the heating element to heat the aerosol forming substrate.

The cavity may comprise a (e.g. circumferential) wall (or walls) and the (tubular) heating element may extend around at least a portion of the wall(s). In this way, the wall may be located between the inner surface of the heating element and an outer surface of the aerosol-forming article. The wall (or walls) of the cavity may be formed from a thermally conductive material (e.g. a metal) to allow heat conduction from the heating element to the aerosol-forming article. Thus, heat may be conducted from the heating element, through the cavity wall (or walls), to the aerosol-forming substrate of an aerosol-forming article received in the cavity.

In some embodiments housing of the device may include a first end for engaging a cap, that is configured for engagement with an aerosol-forming article. Where the device comprises a heater having a heating element, the cap may at least partially enclose the heating element. The cap may be moveable between an open position in which access is provided to the heating element, and a closed position in which the cap at least partially encloses the heating element. The cap may be slideably engaged with the housing of the device, and may be slideable between the open and closed positions. When the cap is engaged with the housing (i.e. the cap in the closed position), a gap may form between the cap and the housing, which may be configured as an air inlet, to facilitate flow of air into the housing. The air entering the housing may be hence, directed underneath the heating element accommodated in the housing.

The cap may define at least a portion of the cavity of the device. That is, the cavity may be fully defined by the cap, or each of the cap and housing may define a portion of the cavity. Where the cap fully defines the cavity, the cap may comprise an aperture for receipt of the heating element into the cavity (when the cap is in the closed position). The cap may comprise an opening to the cavity. The opening may be configured for receipt of at least a portion of an aerosol-forming article. That is, an aerosol-forming article may be inserted through the opening and into the cavity (so as to be engaged with the device).

The cap may be configured such that when an aerosol-forming article is engaged with the device (e.g. received in the cavity), only a portion of the aerosol-forming article is received in the cavity. That is, a portion of the aerosol-forming article (not received in the cavity) may protrude from (i.e. extend beyond) the opening. This (protruding) portion of the aerosol-forming article may be a terminal (e.g. mouth) end of the aerosol-forming article, which may be received in a user's mouth for the purpose of inhaling aerosol formed by the device.

The device may comprise a power source or may be connectable to a power source (e.g. a power source separate to the device). The power source may be electrically connectable to the heater. In that respect, altering (e.g. toggling) the electrical connection of the power source to the heater may affect a state of the heater. For example, toggling the electrical connection of the power source to the heater may toggle the heater between an on state and an off state. The power source may be a power store. For example, the power source may be a battery or rechargeable battery (e.g. a lithium ion battery).

The device may comprise an electrical connection or an input connection (e.g. a USB port, Micro USB port, USB-C port, etc.) disposed at a second end of the housing. The input connection may be configured for connection to an external source of electrical power, such as a mains electrical supply outlet. The input connection may, in some cases, be used as a substitute for an internal power source (e.g. battery or rechargeable battery). That is, the input connection may be electrically connectable to the heater (for providing power to the heater). Hence, in some forms, the input connection may form at least part of the power source of the device. Further, the electrical connection may be configured to provide with the air inlet, to facilitate flow of air into the housing (i.e. underneath the heating element accommodated in the housing).
The power source comprises a rechargeable power source (such as a rechargeable battery), the input connection may be used to charge and recharge the power source.

The device may comprise a user interface (Ul). In some embodiments the UI may include input means to receive operative commands from the user. The input means of the UI may allow the user to control at least one aspect of the operation of the device. In some embodiments the input means may comprise a power button to switch the device between an on state and an off state.

In some embodiments the UI may additionally or alternatively comprise output means to convey information to the user. In some embodiments the output means may comprise a light to indicate a condition of the device (and/or the aerosol-forming article) to the user. The condition of the device (and/or aerosol-forming article) indicated to the user may comprise a condition indicative of the operation of the heater. For example, the condition may comprise whether the heater is in an off state or an on state. In some embodiments, the UI unit may comprise at least one of a button, a display, a touchscreen, a switch, a light, and the like. For example, the output means may comprise one or more (e.g. two, three, four, etc.) light-emitting diodes ("LEDs") that may be located on the housing of the device.

The device may further comprise a puff sensor (e.g. airflow sensor), which form part of the input means of the Ul. The puff sensor may be configured to detect a user drawing on an end (i.e. a terminal (mouth) end) of the aerosol-forming article. The puff sensor may, for example, be a pressure sensor or a microphone. The puff sensor may be configured to produce a signal indicative of a puff state. The signal may be indicative of the user drawing (an aerosol from the aerosol-forming article) such that it is e.g. in the form of a binary signal. Alternatively or additionally, the signal may be indicative of a characteristic of the draw (e.g. a flow rate of the draw, length of time of the draw, etc.).

The device may comprise a controller, or may be connectable to a controller that may be configured to control at least one function of the device. The controller may comprise a microcontroller that may e.g. be mounted on a printed circuit board (PCB). The controller may also comprise a memory, e.g. non-volatile memory. The memory may include instructions, which, when implemented, may cause the controller to perform certain tasks or steps of a method. Where the device comprises an input connection, the controller may be connected to the input connection.

The controller may be configured to control the operation of the heater (and e.g. the heating element). Thus, the controller may be configured to control vaporisation of an aerosol forming part of an aerosol-forming article engaged with the device. The controller may be configured to control the voltage applied by power source to the heater. For example, the controller may be configured to toggle between applying a full output voltage (of the power source) to the heater and applying no voltage to the heater. Alternatively or additionally, the control unit may implement a more complex heater control protocol.

The device may further comprise a voltage regulator to regulate the output voltage supplied by the power source to form a regulated voltage. The regulated voltage may subsequently be applied to the heater.

In some embodiments, where the device comprises a Ul, the controller may be operatively connected to one or more components of the Ul. The controller may be configured to receive command signals from an input means of the Ul. The controller may be configured to control the heater in response to the command signals. For example, the controller may be configured to receive "on" and "off command signals from the UI and, in response, may control the heater so as to be in a corresponding on or off state.

The controller may be configured to send output signals to a component of the Ul. The UI may be configured to convey information to a user, via an output means, in response to such output signals (received from the controller). For example, where the device comprises one or more LEDs, the LEDs may be operatively connected to the controller. Hence, the controller may configured to control the illumination of the LEDs (e.g. in response to an output signal). For example, the controller may be configured to control the illumination of the LEDs according to (e.g. an on or off) state of the heater.

Where the device comprises a sensor (e.g. a puff/airflow sensor), the controller may be operatively connected to the sensor. The controller may be configured to receive a signal from the sensor (e.g. indicative of a condition of the device and/or engaged aerosol-forming article). The controller may be configured to control the heater, or an aspect of the output means, based on the signal from the sensor.

The device may comprise a wireless interface configured to communicate wirelessly (e.g. via Bluetooth (e.g. a Bluetooth low-energy connection) or WiFi) with an external device. Similarly, the input connection may be configured for wired connection to an external device so as to provide communication between the device and the external device.

The external device may be a mobile device. For example, the external device may be a smart phone, tablet, smart watch, or smart car. An application (e.g. app) may be installed on the external device (e.g. mobile device). The application may facilitate communication between the device and the external device via the wired or wireless connection.

The wireless or wired interface may be configured to transfer signals between the external device and the controller of the device. In this respect, the controller may control an aspect of the device in response to a signal received from an external device. Alternatively or additionally, an external device may respond to a signal received from the device (e.g. from the controller of the device).

In a third aspect, there is provided a system (e.g. a smoking substitute system) comprising a device according to the first aspect or the second aspect and an aerosol-forming article. The aerosol-forming article may comprise an aerosol-forming substrate at an upstream end of the aerosol-forming article. Conveniently, the article may be in the form of a smoking substitute article, e.g. heated tobacco (HT) consumable (also known as a heat-not-burn (HNB) consumable).

As used herein, the terms "upstream" and "downstream" are intended to refer to the flow direction of the vapour/aerosol i.e. with the downstream end of the article/consumable being the mouth end or outlet where the aerosol exits the consumable for inhalation by the user. The upstream end of the article/consumable is the opposing end to the downstream end.

The aerosol-forming substrate is capable of being heated to release at least one volatile compound that can form an aerosol. The aerosol-forming substrate may be located at the upstream end of the article/consumable.

In order to generate an aerosol, the aerosol-forming substrate comprises at least one volatile compound that is intended to be vaporised/aerosolised and that may provide the user with a recreational and/or medicinal effect when inhaled. Suitable chemical and/or physiologically active volatile compounds include the group consisting of: nicotine, cocaine, caffeine, opiates and opoids, cathine and cathinone, kavalactones, mysticin, beta-carboline alkaloids, salvinorin A together with any combinations, functional equivalents to, and/or synthetic alternatives of the foregoing.

The aerosol-forming substrate may comprise plant material. The plant material may comprise least one plant material selected from the list including *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), Opium poppy, *Passiflora incamata* (Passionflower), *Pedicularis densiflora* (Indian Warrior), *Pedicularis groenlandica* (Elephant's Head), *Salvia divinorum, Salvia dorrii* (Tobacco Sage), Salvia species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava) together with any combinations, functional equivalents to, and/or synthetic alternatives of the foregoing.

The plant material may be tobacco. Any type of tobacco may be used. This includes, but is not limited to, flue-cured tobacco, burley tobacco, Maryland Tobacco, dark-air cured tobacco, oriental tobacco, dark-fired tobacco, perique tobacco and rustica tobacco. This also includes blends of the above mentioned tobaccos.

The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon).

The aerosol-forming substrate may comprise a gathered sheet of homogenised (e.g. paper/slurry recon) tobacco or gathered shreds/strips formed from such a sheet.

The aerosol-forming substrate may comprise one or more additives selected from humectants, flavourants, fillers, aqueous/non-aqueous solvents and binders.

The flavourant may be provided in solid or liquid form. It may include menthol, liquorice, chocolate, fruit flavour (including e.g. citrus, cherry etc.), vanilla, spice (e.g. ginger, cinnamon) and tobacco flavour. The flavourant may be evenly dispersed throughout the aerosol-forming substrate or may be provided in isolated locations and/or varying concentrations throughout the aerosol-forming substrate.

The aerosol-forming substrate may be formed in a substantially cylindrical shape such that the article/consumable resembles a conventional cigarette. It may have a diameter of between 5 and 10mm e.g. between 6 and 9mm or 6 and 8mm e.g. around 7 mm. It may have an axial length of between 10 and 15mm e.g. between 11 and 14mm such as around 12 or 13mm.

The article/consumable may comprise at least one filter element. There may be a terminal filter element at the downstream/mouth end of the article/consumable.

The or at least one of the filter element(s) (e.g. the terminal filter element) may be comprised of cellulose acetate or polypropylene tow. The at least one filter element (e.g. the terminal filter element) may be comprised of activated charcoal. The at least one filter element (e.g. the terminal element) may be comprised of paper. The or each filter element may be at least partly (e.g. entirely) circumscribed with a plug wrap e.g. a paper plug wrap.

The terminal filter element (at the downstream end of the article/consumable) may be joined to the upstream elements forming the article/consumable by a circumscribing tipping layer e.g. a tipping paper layer. The tipping paper may have an axial length longer than the axial length of the terminal filter element such that the tipping paper completely circumscribes the terminal filter element plus the wrapping layer surrounding any adjacent upstream element.

In some embodiments, the article/consumable may comprise an aerosol-cooling element which is adapted to cool the aerosol generated from the aerosol-forming substrate (by heat exchange) before being inhaled by the user.

The article/consumable may comprise a spacer element that defines a space or cavity between the aerosol-forming substrate and the downstream end of the consumable. The spacer element may comprise a cardboard tube. The spacer element may be circumscribed by the (paper) wrapping layer.

The invention includes the combination of the aspects and preferred features described except where such a combination is clearly impermissible or expressly avoided.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### SUMMARY OF THE FIGURES

So that the invention may be understood, and so that further aspects and features thereof may be appreciated, embodiments illustrating the principles of the invention will now be discussed in further detail with reference to the accompanying figures, in which:
Figure 1A is a schematic of a smoking substitute system;
Figure 1B is a schematic of a variation of the smoking substitute system of Figure 1A;
Figure 2A is a front view of a first embodiment of a smoking substitute system with the consumable engaged with the device;
Figure 2B is a front view of the first embodiment of the smoking substitute device;
Figure 2C is a section view of the consumable of the first embodiment of the smoking substitute system;
Figure 2D is a detailed view of the first end of the smoking substitute device;
Figure 2E is a sectional view of the second embodiment of the smoking substitute system;
Figure 3 is detailed view of a first end of a third embodiment of the smoking substitute device; and
Figure 4 is a front view of the fourth embodiment of the smoking substitute device.

### DETAILED DESCRIPTION OF THE INVENTION

Aspects and embodiments of the present invention will now be discussed with reference to the accompanying figures. Further aspects and embodiments will be apparent to those skilled in the art.

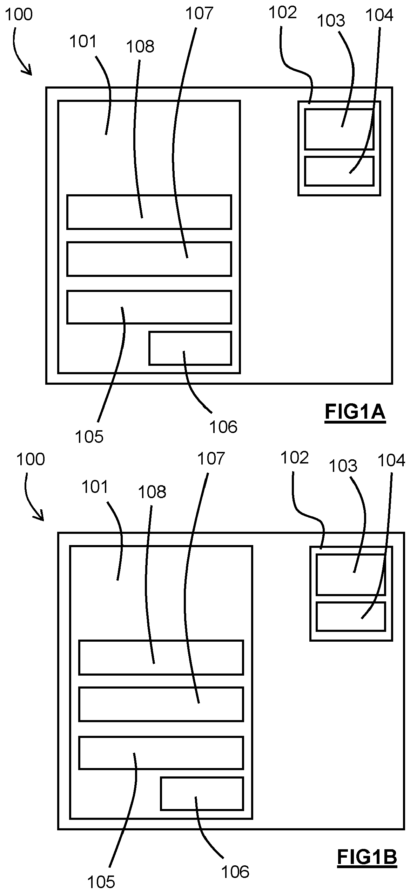

Figure 1A is a schematic providing a general overview of a smoking substitute system 100. The system 100 includes a substitute smoking device 101 and an aerosol-forming article in the form of a consumable 102, which comprises an aerosol former 103. The system is configured to vaporise the aerosol former by heating the aerosol former 103 (so as to form a vapour/aerosol for inhalation by a user).

In the illustrated system, the heater 104 forms part of the consumable 102 and is configured to heat the aerosol former 103. In this variation, the heater 104 is electrically connectable to the power source 105, for example, when the consumable 102 is engaged with the device 101. Heat from the heater 104 vaporises the aerosol former 103 to produce a vapour. The vapour subsequently condenses to form an aerosol, which is ultimately inhaled by the user.

The system 100 further comprises a power source 105 that forms part of the device 101. In other embodiments the power source 105 may be external to (but connectable to) the device 101. The power source 105 is electrically connectable to the heater 104 such that it is able to supply power to the heater 104 (i.e. for the purpose of heating the aerosol former 103). Thus, control of the electrical connection of the power source 105 to the heater 104 provides control of the state of the heater 104. The power source 105 may be a power store, for example a battery or rechargeable battery (e.g. a lithium ion battery).

The system 100 further comprises an I/O module comprising an electrical connection106 (e.g. in the form of a USB port, Micro USB port, USB-C port, etc.). The electrical connection106 is configured for connection to an external source of electrical power, e.g. a mains electrical supply outlet. The electrical connection 106 may be used in substitution for the power source 105. That is the electrical connection 106 may be electrically connectable to the heater 104 so as to supply electricity to the heater 104. In such embodiments, the device may not include a power source, and the power source of the system may instead comprise the electrical connection 106 and an external source of electrical power.

In some embodiments, the electrical connection 106 may be used to charge and recharge the power source 105 where the power source 105 includes a rechargeable battery.

In some embodiments, the electrical connection 106, may be configured to provide an air inlet, to facilitate flow of air into the housing i.e. underneath the heater 104 (thus the heating element).

The system 100 also comprises a user interface (Ul) 107. Although not shown, the Ul 107 may include input means to receive commands from a user. The input means of the UI 107 allows the user to control at least one aspect of the operation of the system 100. The input means may, for example, be in the form of a button, touchscreen, switch, microphone, etc.

The UI 107 also comprises output means to convey information to the user. The output means may, for example, comprise lights (e.g. LEDs), a display screen, speaker, vibration generator, etc.

The system 100 further comprises a controller 108 that is configured to control at least one function of the device 101. In the illustrated embodiment, the controller 108 is a component of the device 101, but in other embodiments may be separate from (but connectable to) the device 101. The controller 108 is configured to control the operation of the heater 104 and, for example, may be configured to control the voltage applied from the power source 105 to the heater 104. The controller 108 may be configured to toggle the supply of power to the heater 104 between an on state, in which the full output voltage of the power source 105 is applied to the heater 104, and an off state, in which the no voltage is applied to the heater 104.

Although not shown, the system 100 may also comprise a voltage regulator to regulate the output voltage from the power source 105 to form a regulated voltage. The regulated voltage may then be applied to the heater 104.

In addition to being connected to the heater 104, the controller 108 is operatively connected to the UI 107. Thus, the controller 108 may receive an input signal from the input means of the UI 107. Similarly, the controller 108 may transmit output signals to the UI 107. In response, the output means of the UI 107 may convey information, based on the output signals, to a user. The controller also comprises a memory 109, which is a non-volatile memory. The memory 109 includes instructions, which, when implemented, cause the controller to perform certain tasks or steps of a method.

Figure 1B is a schematic showing a variation of the system 100 of Figure 1A. In the system 100' of Figure 1B, the heater 104 forms part of the device 101, rather than the consumable 102. In this variation, the heater 104 is electrically connected to the power source 105.

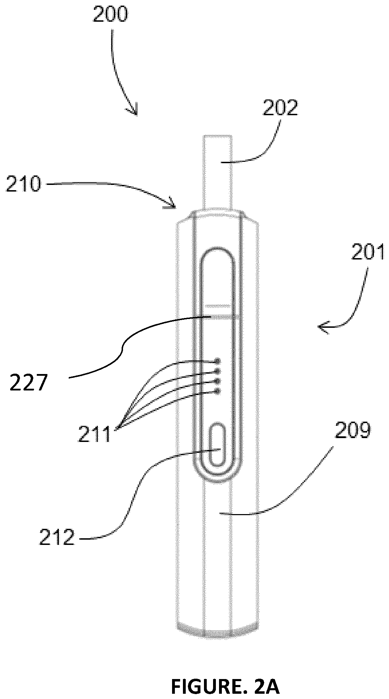

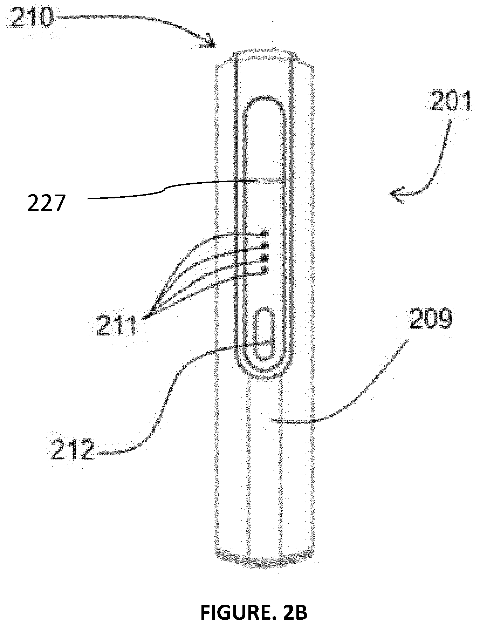

Figure 2A illustrates a heated-tobacco (HT) smoking substitute system 200. The system 200 is an example of the systems 100, 100' described in relation to Figures 1A or 1B. System 200 includes a smoking substitute device 201 and an HT consumable 202. The description of Figures 1A and 1B above is applicable to the system 200 of Figures 2A and 2B, and will thus not be repeated.

Figure. 2B illustrates the device 201 of the smoking substitute system 200. The device 201 is configured to receive a consumable 202 (as seen in figure. 2A), which may facilitate in generating aerosol under operating conditions of the device 201.

The device 201 comprises a housing 209 and cap 210. In use, the cap 210 is engageable at a first end of the housing 209. Although not apparent from the figures, the cap 210 is moveable relative to the housing 209. In particular, the cap 210 is slideable and can slide along a longitudinal axis of the housing 209.

In some embodiments, the cap 210 is movable along a longitudinal axis of the housing 209.

The device 201 comprises an output means (forming part of the UI of the device 201) in the form of a plurality of light-emitting diodes (LEDs) 211 arranged linearly along the longitudinal axis of the device 201 and on an outer surface of the housing 209 of the device 201. A button 212 is also arranged on an outer surface of the housing 209 of the device 201 and is axially spaced (i.e. along the longitudinal axis) from the plurality of LEDs 211.

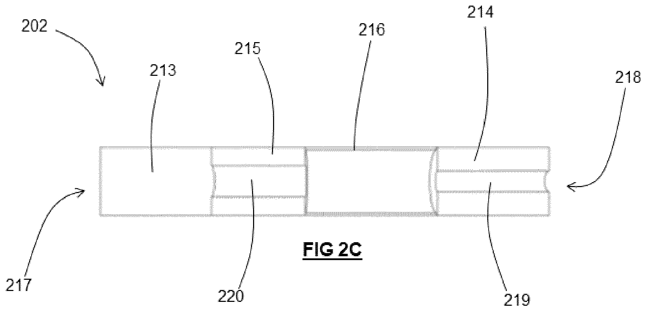

Figure 2C show a detailed section view of the consumable of 202 of the system 200. The consumable 202 generally resembles a cigarette. In that respect, the consumable 202 has a generally cylindrical form with a diameter of 7 mm and an axial length of 70 mm. The consumable 202 comprises an aerosol forming substrate 213, a terminal filter element 214, an upstream filter element 215 and a spacer element 216. In other embodiments, the consumable may further comprise a cooling element. A cooling element may exchange heat with vapour that is formed by the aerosol-forming substrate 213 in order to cool the vapour so as to facilitate condensation of the vapour.

The aerosol-forming substrate 213 is substantially cylindrical and is located at an upstream end 217 of the consumable 202, and comprises the aerosol former of the system 200. In that respect, the aerosol forming substrate 213 is configured to be heated by the device 201 to release a vapour. The released vapour is subsequently entrained in an airflow flowing through the aerosol-forming substrate 213. The airflow is produced by the action of the user drawing on a downstream 218 (i.e. terminal or mouth) end of the consumable 202.

In the present embodiment, the aerosol forming substrate 213 comprises tobacco material that may, for example, include any suitable parts of the tobacco plant (e.g. leaves, stems, roots, bark, seeds and flowers). The tobacco may comprise one or more of leaf tobacco, stem tobacco, tobacco powder, tobacco dust, tobacco derivatives, expanded tobacco, homogenised tobacco, shredded tobacco, extruded tobacco, cut rag tobacco and/or reconstituted tobacco (e.g. slurry recon or paper recon). For example, the aerosol-forming substrate 213 may comprise a gathered sheet of homogenised (e.g. paper/slurry recon) tobacco or gathered shreds/strips formed from such a sheet.

In order to generate an aerosol, the aerosol forming substrate 213 comprises at least one volatile compound that is intended to be vaporised/aerosolised and that may provide the user with a recreational and/or medicinal effect when inhaled. The aerosol-forming substrate 213 may further comprise one or more additives. For example, such additives may be in the form of humectants (e.g. propylene glycol and/or vegetable glycerine), flavourants, fillers, aqueous/non-aqueous solvents and/or binders.

The terminal filter element 214 is also substantially cylindrical, and is located downstream of the aerosol forming substrate 213 at the downstream end 218 of the consumable 202. The terminal filter element 214 is in the form of a hollow bore filter element having a bore 219 (e.g. for airflow) formed therethrough. The diameter of the bore 219 is 2 mm. The terminal filter element 214 is formed of a porous (e.g. monoacetate) filter material. As set forth above, the downstream end 218 of the consumable 202 (i.e. where the terminal filter 214 is located) forms a mouthpiece portion of the consumable 202 upon which the user draws. Airflow is drawn from the upstream end 217, thorough the components of the consumable 202, and out of the downstream end 218. The airflow is driven by the user drawing on the downstream end 218 (i.e. the mouthpiece portion) of the consumable 202.

The upstream filter element 215 is located axially adjacent to the aerosol-forming substrate 213, between the aerosol-forming substrate 213 and the terminal filter element 214. Like the terminal filter 214, the upstream filter element 215 is in the form of a hollow bore filter element, such that it has a bore 220 extending axially therethrough. In this way, the upstream filter 215 may act as an airflow restrictor. The upstream filter element 215 is formed of a porous (e.g. monoacetate) filter material. The bore 220 of the upstream filter element 215 has a larger diameter (3 mm) than the terminal filter element 214.

The spacer 216 is in the form of a cardboard tube, which defines a cavity or chamber between the upstream filter element 215 and the terminal filter element 214. The spacer 216 acts to allow both cooling and mixing of the vapour/aerosol from the aerosol-forming substrate 213. The spacer has an external diameter of 7 mm and an axial length of 14mm.

Although not apparent from the figure, the aerosol-forming substrate 213, upstream filter 215 and spacer 216 are circumscribed by a paper wrapping layer. The terminal filter 214 is circumscribed by a tipping layer that also circumscribes a portion of the paper wrapping layer (so as to connect the terminal filter 214 to the remaining components of the consumable 202). The upstream filter 215 and terminal filter 214 are circumscribed by further wrapping layers in the form of plug wraps.

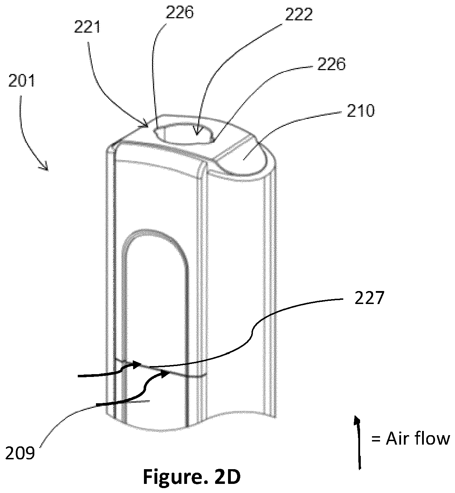

Returning now to the device 201, Figure 2D illustrates a detailed view of the first end of the device 201 that is configured to engage with the cap 210. The cap 210 and the housing 209 are engaged by the mechanism, wherein the mechanism is at least one of a snap fit mechanism, a magnetic lock mechanism or any other mechanism that serves the purpose engaging the cap 210 with the housing 209. The cap 210 of the device 201 includes an opening 221 to an internal cavity 222 (more apparent from Figure 2D) defined by the cap 210. The opening 221 and the cavity 222 are formed so as to receive at least a portion of the consumable 202. During engagement of the consumable 202 with the device 201, a portion of the consumable 202 is received through the opening 221 and into the cavity 222. After engagement (see Figure 2B), the downstream end 218 of the consumable 202 protrudes from the opening 221 and thus also protrudes from the device 201. The opening 221 includes laterally disposed notches 226. When a consumable 202 is received in the opening 221, these notches 226 remain open and could, for example, be used for retaining a cover in order to cover the end of the device 201.

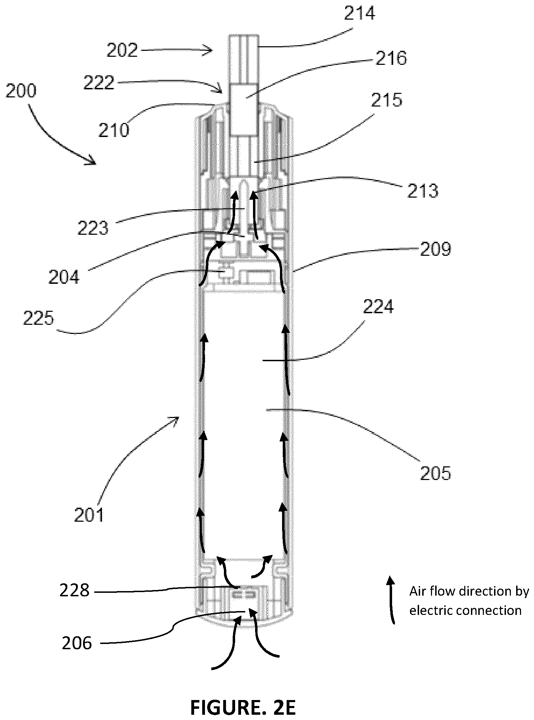

Figure 2E shows a cross section through a central longitudinal plane through the device 201. The device 201 is shown with the consumable 202 engaged therewith.

The device 201 comprises a heater 204 comprising heating element 223. The heater 204 forms part of the housing 209 of the device 201 and is rigidly mounted to the housing 209. In the illustrated embodiment, the heater 204 is a rod heater with a heating element 223 having a circular transverse profile. In other embodiments the heater may be in the form of a blade heater (e.g. heating element with a rectangular transverse profile) or a tube heater (e.g. heating element with a tubular form).

In an embodiment, the heating element 223 of the heater 204 may be configured to penetrate through at least a portion of the consumable 202, so as the transfer heat to the consumable 202 to generate aerosol.

The heating element 223 of the heater 204 projects from an internal base of the cavity 222 along a longitudinal axis towards the opening 221. As is apparent from the figure, the length (i.e. along the longitudinal axis) of the heating element is less than a depth of the cavity 222. In this way, the heating element 223 does not protrude from or extend beyond the opening 221.

When the consumable 202 is received in the cavity 222 (as is shown in Figure 2E), the heating element 223 penetrates the aerosol-forming substrate 213 of the consumable 202. In particular, the heating element 223 extends for nearly the entire axial length of the aerosol-forming substrate 213 when inserted therein. Thus, when the heater 204 is activated, heat is transferred radially from an outer circumferential surface the heating element 223 to the aerosol-forming substrate 213.

Returning back to figure 2B and 2D which illustrates the device 201, with the cap 210 and the housing 209 engaged with one another, in order to enclose at least a portion of the heating element 223 of the heater 204 (seen in figure 2D). The cap 210 and the housing 209 may be configured to define a gap 227 between the cap 210 and the housing 209, upon engagement of the cap 210 and the housing 209. The cap 210 and the housing 209 may be engaged with an interference fit, so as to form the gap 227 between the cap 210 and the housing 209.

In the illustrated embodiment of Figure 2D, the gap 227 defined between the cap 210 and the housing 209 upon engagement, may be configured as an air inlet, to facilitate flow of air into the housing 209. Due to such an engagement of the cap 210 and the housing 209, the air inlet 227 (thus the gap), may be configured to extend in a direction transverse to longitudinal axis of the housing 209, e.g. extend linearly and transversely in a major surface of the housing 209, with respect to longitudinal axis of the housing 209. Further, the air inlet 227 may be configured to facilitate flow of air adjacent to the heating element 223. Furthermore, the air inlet 227 may configured to facilitate flow of air towards a base of the heating element, e.g. underneath the heating element 223 of the heater 204 residing within the housing 209.

In an embodiment, and referring to figures 2B, the housing 209 of the device 201, may be an elongated member, with a length of the housing 201 greater than thickness of the housing 209. Thus, the major surface of the housing 209 may be at least one of a front face and a rear face of the housing 209, which possess surface area greater than that of the side surfaces. Now, referring back to Figure. 2E, the device 201 comprises an electronics cavity 224. A power source, in the form of a rechargeable battery 205 (a lithium ion battery), is located in electronics cavity 224.

The device 201 includes an electrical connection 206 (i.e. forming part of an IO module of the device 201) in the form of a Universal Serial Bus port (USB port), disposed at a second end of the housing 209. In an embodiment, the second end may be a bottom end. The connector may alternatively be, for example, a micro-USB port or a USB-C port for examples. The electrical connection 206 may be used to recharge the rechargeable battery 205. In the illustrated embodiment as shown in Figure 2E, the electrical connection 206 may be configured to provide with an air inlet 228, to facilitate flow of air into the housing 209. The air inlet 228 provided in the electrical connection 206, may be an aperture or a slit configured at an end wall (not shown) of the electrical connection 206. The air may enter through the air inlet 228 provided in the electrical connection 206 and may flow through a substantial length of the housing 209. Also, the air entering the housing 209, flows towards the base of the heating element 223, e.g. underneath the heating element 223 of the heater 204 (indicated with arrows). Thus, the air inlet in the electrical connection 206 may facilitate in flow of air underneath the heating element 223, to improve aerosol formation.

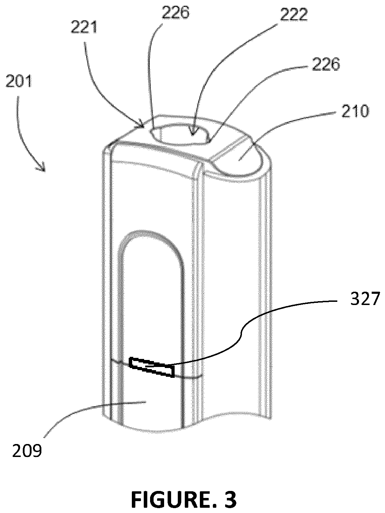

Referring to figure. 3, illustrates a detailed view of a third embodiment of the first end of the housing 209. As shown in Figure. 3, the cap 210 of the device 201 is provided with a notch 327, e.g. configured with a slit or a provision, which is configured to act as an air inlet. The notch 327 is formed on an edge of the cap 210. The notch 327 is configured to facilitate flow of air underneath the heating element 223 of the heater 204, accommodated in the housing 209.

In some embodiments, the cap 210 may be configured with through holes or apertures on one of the major surface, to facilitate flow of air into the housing 209 and underneath the heating element 223.

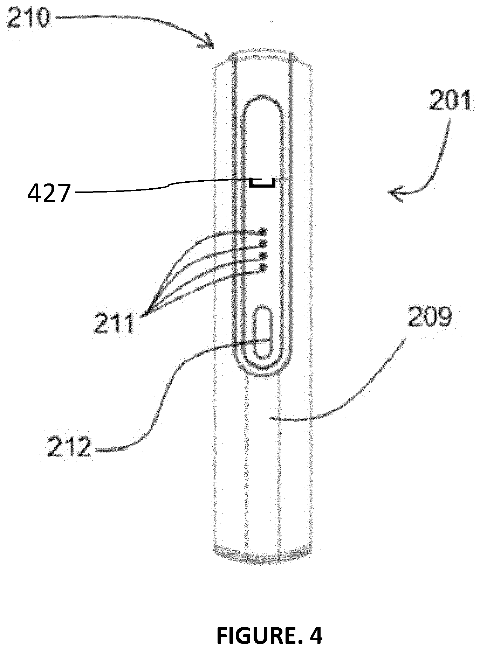

Referring to figure. 4 illustrates a front view of the device 201. In this embodiment, the housing 209 of the device is defined with a notch 427. The notch 427 is defined at an interface of the cap 210 and the housing 209. The notch 427 is formed on an edge of the housing 209, and act as the air inlet. The notch 427 is configured to facilitate flow of air underneath the heating element 223 accommodated in the housing 209.

In an embodiment, the air from the surroundings may be drawn through either of the gap 227 defined between the cap 210 and the housing 209, and the air inlet 228 provided in the USB port 206, into the housing 209 as the user draws aerosol through the consumable 202. The aerosol may be formed due to interaction of the consumable 202 with the heat generated by the heating element 223. Upon drawing the aerosol, pressure developed inside the housing 209 of the device 201 decreases due to which, the air from the surroundings may enters into the housing 209 (i.e. underneath the heating element 223), through the air inlets 227, 228 configured in the device 201. The flow of air into the housing 209 (i.e. underneath the heating element 223), mixes with the heat generated from the heating element 223, which facilitates in improving aerosol generation and total particulate matter (TPM) output of the aerosol.

In some embodiments, the air inlets configured in the device 201, account to minimum form factor changes and do not interfere with the design of the device 201. Since, the air inlets are defined within or between essential components (e.g. the cap 210, the housing 209, the electrical connection 206) of the device 201, this feature may facilitate in defining the air inlets, without affecting or altering the profile of the device 201.

The device 201 includes a controller (not shown) located in the electronics cavity 224. The controller comprises a microcontroller mounted on a printed circuit board (PCB). The USB port 206 is also connected to the controller 208 (i.e. connected to the PCB and microcontroller).

The controller 208 is configured to control at least one function of the device 202. For example, the controller 208 is configured to control the operation of the heater 204. Such control of the operation of the heater 204 may be accomplished by the controller toggling the electrical connection of the rechargeable battery 205 to the heater 204. For example, the controller 208 is configured to control the heater 204 in response to a user depressing the button 212. Depressing the button 212 may cause the controller to allow a voltage (from the rechargeable battery 205) to be applied to the heater 204 (so as to cause the heating element 223 to be heated).

The controller is also configured to control the LEDs 211 in response to (e.g. a detected) a condition of the device 201 or the consumable 202. For example, the controller may control the LEDs to indicate whether the device 201 is in an on state or an off state (e.g. one or more of the LEDs may be illuminated by the controller when the device is in an on state).

The device 201 comprises a further input means (i.e. in addition to the button 212) in the form of a puff sensor 225. The puff sensor 225 is configured to detect a user drawing (i.e. inhaling) at the downstream end 218 of the consumable 202. The puff sensor 225 may, for example, be in the form of a pressure sensor, flowmeter or a microphone. The puff sensor 225 is operatively connected to the controller 208 in the electronics cavity 224, such that a signal from the puff sensor 225, indicative of a puff state (i.e. drawing or not drawing), forms an input to the controller 208 (and can thus be responded to by the controller 208).

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A smoking substitute device (101, 201), comprising:
a housing (209); and
a cap (210); wherein
a heating element (223) extends from a first end of the housing (209), the first end of the housing being configured for engagement with the cap (210);
the cap (210) includes an opening (221) to an internal cavity (222) for receiving a part of a consumable (202);
**characterised in that**
the cap (210) is configured to engage with the housing (209) and thereby define an air inlet (227) between the cap (210) and the housing (209);
wherein the air inlet (227) extends in a direction transverse to the longitudinal axis of the housing (209) to facilitate an airflow to enter into the housing and through the consumable when a user draws on the consumable (202).

2. The smoking substitute (101, 201) device according to any one of the preceding claims, wherein the air inlet (227) is located adjacent to a base of the heating element (223) of the housing (209) to facilitate the airflow to flow towards the base of the heating element (223).

3. The smoking substitute device (101, 201) according to any one of the preceding claims, wherein the air inlet (227) is defined by a gap formed between the cap (210) and the housing (209) when the cap (210) is engaged with the housing (209).

4. The smoking substitute device (101, 201) according to any one of the preceding claims, wherein the cap (210) and/or housing (209) comprises a notch (327, 427) formed on a respective edge of the cap (210) and/or housing (209), wherein the notch (327, 427) on the cap (210) and/or housing (209) forms the air inlet (227).

5. The smoking substitute device (101, 201) according to any one of the preceding claims, wherein the air inlet (227) comprises a slit or a through hole.

6. The smoking substitute device (101, 201) according to any one of the preceding claims, wherein the smoking substitute device (101, 201) comprises a Heat Not Burn (HNB) device.

7. A smoking substitute system (100, 200), comprising:
i) a smoking substitute device (101, 201) according to any one of the claims 1 to 6; and
ii) an aerosol-forming article for use with the device (101, 201).

## Patentansprüche

1. Rauchersatzvorrichtung (101, 201), umfassend:
ein Gehäuse (209); und
eine Kappe (210); wobei
sich ein Heizelement (223) von einem ersten Ende des Gehäuses (209) aus erstreckt, wobei das erste Ende des Gehäuses zum Eingriff mit der Kappe (210) ausgelegt ist;
wobei die Kappe (210) eine Öffnung (221) zu einem inneren Hohlraum (222) zum Aufnehmen eines Teils einer Verbrauchsware (202) umfasst;
**dadurch gekennzeichnet, dass**:
die Kappe (210) ausgelegt ist, um mit dem Gehäuse (209) in Eingriff zu sein und dadurch einen Lufteinlass (227) zwischen der Kappe (210) und dem Gehäuse (209) zu definieren;
wobei sich der Lufteinlass (227) in eine Richtung quer zur Längsachse des Gehäuses (209) erstreckt, um zu erleichtern, dass eine Luftströmung in das Gehäuse und durch die Verbrauchsware strömt, wenn ein Benutzer von der Verbrauchsware (202) einen Zug nimmt.

2. Rauchersatzvorrichtung (101, 201) nach einem der vorangegangenen Ansprüche, wobei der Lufteinlass (227) benachbart zu einer Basis des Heizelements (223) des Gehäuses (209) angeordnet ist, um zu erleichtern, dass die Luftströmung in Richtung der Basis des Heizelements (223) strömt.

3. Rauchersatzvorrichtung (101, 201) nach einem der vorangegangenen Ansprüche, wobei der Lufteinlass (227) durch einen Spalt definiert ist, der zwischen der Kappe (210) und dem Gehäuse (209) ausgebildet ist, wenn die Kappe (210) mit dem Gehäuse (209) in Eingriff steht.

4. Rauchersatzvorrichtung (101, 201) nach einem der vorangegangenen Ansprüche, wobei die Kappe (210) und/oder das Gehäuse (209) eine Vertiefung (327, 427) umfasst, die auf einem entsprechenden Rand der Kappe (210) und/oder des Gehäuses (209) ausgebildet ist, wobei die Vertiefung (327, 427) auf der Kappe (210) und/oder dem Gehäuse (209) den Lufteinlass (227) ausbildet.

5. Rauchersatzvorrichtung (101, 201) nach einem der vorangegangenen Ansprüche, wobei der Lufteinlass (227) einen Schlitz oder ein Durchgangsloch umfasst.

6. Rauchersatzvorrichtung (101, 201) nach einem der vorangegangenen Ansprüche, wobei die Rauchersatzvorrichtung (101, 201) eine Erhitzen-anstatt-Verbrennen-(HNB-) Vorrichtung umfasst.

7. Rauchersatzsystem (100, 200), umfassend:
i) eine Rauchersatzvorrichtung (101, 201) nach einem der Ansprüche 1 bis 6; und
ii) einen aerosolbildenden Artikel zur Verwendung mit der Vorrichtung (101, 201).

## Revendications

1. Dispositif à fumer de substitution (201), comprenant :
un logement (209) ; et
un capuchon (210) ; dans lequel
un élément chauffant (223) s'étend à partir d'une première extrémité du logement (209), la première extrémité du logement étant configurée pour venir en prise avec le capuchon (210) ;
le capuchon (210) inclut une ouverture (221) vers une cavité interne (222) pour recevoir une partie d'un consommable (202) ;
**caractérisé en ce que**
le capuchon (210) est configuré pour venir en prise avec le logement (209) et définir ainsi une entrée d'air (227) entre le capuchon (210) et le logement (209) ;
dans lequel l'entrée d'air (227) s'étend dans une direction transversale à l'axe longitudinal du logement (209) pour faciliter une entrée d'un flux d'air dans le logement et à travers le consommable lorsqu'un utilisateur utilise le consommable (202).

2. Dispositif à fumer de substitution (101, 201) selon l'une quelconque des revendications précédentes, dans lequel l'entrée d'air (227) est située adjacente à une base de l'élément chauffant (223) du logement (209) pour faciliter l'écoulement du flux d'air vers la base de l'élément chauffant (223).

3. Dispositif à fumer de substitution (101, 201) selon l'une quelconque des revendications précédentes, dans lequel l'entrée d'air (227) est définie par un espace formé entre le capuchon (210) et le logement (209) lorsque le capuchon (210) est mis en prise avec le logement (209).

4. Dispositif à fumer de substitution (101, 201) selon l'une quelconque des revendications précédentes, dans lequel le capuchon (210) et/ou le logement (209) comprend une encoche (327, 427) formée sur un bord respectif du capuchon (210) et/ou du logement (209), dans lequel l'encoche (327, 427) sur le capuchon (210) et/ou le logement (209) forme l'entrée d'air (227).

5. Dispositif à fumer de substitution (101, 201) selon l'une quelconque des revendications précédentes, dans lequel l'entrée d'air (227) comprend une fente ou un trou traversant.

6. Dispositif à fumer de substitution (101, 201) selon l'une quelconque des revendications précédentes, dans lequel le dispositif à fumer de substitution (101, 201) comprend un dispositif chauffé sans combustion (HNB).

7. Système à fumer de substitution (100, 200), comprenant :
un dispositif à fumer de substitution (101, 201) selon l'une quelconque des revendications 1 à 6 ; et
ii) un article de formation d'aérosol à utiliser avec le dispositif (101, 201).
